(19) Europäisches Patentamt
European Patent Office
Office européen des brevets

(11) EP 2 722 043 B1

(12) **EUROPEAN PATENT SPECIFICATION**

(45) Date of publication and mention of the grant of the patent:
**14.12.2016 Bulletin 2016/50**

(51) Int Cl.:
*A61K 31/365* (2006.01) *A61K 31/375* (2006.01)
*A61K 8/00* (2006.01)

(21) Application number: **13004980.2**

(22) Date of filing: **17.10.2013**

(54) **Ex vivo process for stimulating hyaluronic acid production using 3-O-alkyl ascorbic acid derivatives**

Ex vivo Verfahren zur Stimulierung der Hyaluronsäureproduktion mit 3-O-Alkyl-Ascorbinsäurederivaten

Procédé ex vivo permettant de stimuler la production d'acide hyaluronique avec des dérivés d'acide ascorbique 3-O-alkyles

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.10.2012 US 201261715376 P**

(43) Date of publication of application:
**23.04.2014 Bulletin 2014/17**

(73) Proprietor: **Corum Inc.**
**Neihu Dist ,Taipei City 114 (TW)**

(72) Inventors:
- **Lin, Wei-Yu**
  **114 Taipei City (TW)**
- **Wang, Ssu-Ching**
  **114 Taipei City (TW)**
- **Wu, Pei-Hsuan**
  **114 Taipei City (TW)**
- **Yi, Siao-Huei**
  **114 Taipei City (TW)**
- **Yao, Jun-Yi**
  **114 Taipei City (TW)**

(74) Representative: **Zeitler Volpert Kandlbinder Patent- und Rechtsanwälte Partnerschaft mbB**
**Herrnstrasse 44**
**80539 München (DE)**

(56) References cited:
**EP-A2- 1 264 593 JP-A- 2008 105 985**

Remarks:
The file contains technical information submitted after the application was filed and not included in this specification

EP 2 722 043 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The present invention is generally related to an ex vivo process for stimulating the production of hyaluronic acid, and more particularly to an ex vivo process for stimulating the production of hyaluronic acid with 3-O-alkyl ascorbic acid derivatives and the composition thereof.

## 2. Description of the Prior Art

Hyaluronic Acid (HA), also called Hyaluronan or sodium hyaluronate, is a naturally occurring, highly hydrated linear polymer composed of the repeating disaccharide D-glucuronic acid-beta-1, 3-N-acetylglucosamine-beta-1,4. Molecular weight of HA is between 1000 kDa - 10000 kDa. It is found in large concentrations in the extracellular matrix (ECM), which is the fluid-filled space between cells. HA locks moisture into the ECM, keeping collagen and elastin moist and promoting a youthful appearance.

HA is widely used in therapeutics and cosmetology. The most popular source to obtain HA is as the following: animal organ extraction, microorganism fermentation, and chemical synthesis. All the mentioned sources of HA are extrinsic to human body, so that the mentioned HA can be possibly dangerous to human body and cannot stay long time in human body.

EP 1 264 593 A2 relates to a method of preventing darkening of the skin comprising inhibiting melanization of melanin monomers caused by irradiating the melanin monomers by ultraviolet rays.

JP 2008-105985 A relates to a hyaluronic acid production promoter, a skin care preparation for external use, a bathing agent and food and drink.

In view of the above matter, developing a novel ex vivo process and a novel composition for ex vivo stimulating the production of hyaluronic acid having the advantage of high stimulating yield of HA is an important task for the industry.

## SUMMARY OF THE INVENTION

In light of the above background, in order to fulfill the requirements of the industry, the present invention provides a novel ex vivo process for stimulating the production of hyaluronic acid having the advantage of high stimulating yield of hyaluronic acid, so that the ex vivo production of hyaluronic acid can be approached.

One object of the present invention is to provide an ex vivo process for stimulating the production of hyaluronic acid.

Accordingly, the present invention discloses an ex vivo process for stimulating the production of hyaluronic acid. The mentioned ex vivo process for stimulating the production of hyaluronic acid comprises a step of reacting fibroblast cell with 3-O-alkyl ascorbic acid derivatives. According to this invention, through ex vivo stimulating by 3-O-alkyl ascorbic acid derivatives, the ex vivo production of hyaluronic acid can be approached more efficiently and more safety. Preferably, according to this invention, the ex vivo production of hyaluronic acid can be stimulated efficiently by small amount of 3-O-alkyl ascorbic acid derivatives.

According to the invention, the ex vivo process for stimulating the production of hyaluronic acid can employ the 3-O-alkyl ascorbic acid derivatives with the following formula, wherein R1 is H atom and R2 is C2-C10 alkyl group, to stimulate fibroblast cell to prepare hyaluronic acid.

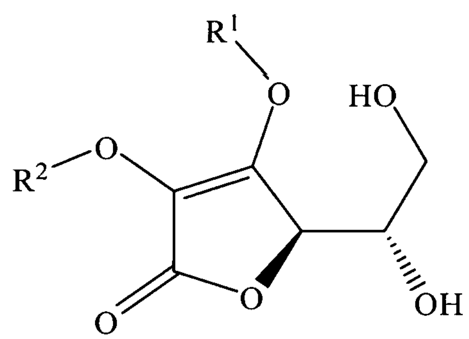

In one preferred embodiment of this invention, the ex vivo process for stimulating the production of hyaluronic acid can employ the 3-O-alkyl ascorbic acid derivatives with the concentration of 7500ppm - 1.25ppm to stimulate fibroblast cell to prepare hyaluronic acid.

In one preferred embodiment of this invention, the ex vivo process for stimulating the production of hyaluronic acid can employ 3-O-alkyl ascorbic acid to stimulate fibroblast cell to prepare hyaluronic acid.

In one preferred embodiment of this invention, the ex vivo process for stimulating the production of hyaluronic acid can employ 3-O-ethyl ascorbic acid to stimulate fibroblast cell to prepare hyaluronic acid.

In one preferred embodiment of this invention, the 3-O-alkyl ascorbic acid derivatives in the composition for ex vivo stimulating the production of hyaluronic acid can be 3-O-ethyl ascorbic acid.

## BRIEF DESCRIPTION OF THE DRAWINGS

Figure 1 presents the analysis diagram of the produced concentration of hyaluronic acid with mouse fibroblast (3T3) stimulated by 3-O-alkyl ascorbic acid derivatives with the ex vivo process according to this specification and measured in Echelon HA-ELISA;

Figure 2 presents the analysis diagram of the produced concentration of hyaluronic acid with human fibroblast (HS68) stimulated by 3-O-alkyl ascorbic acid derivatives with the ex vivo process according to this specification and measured in Echelon HA-ELISA;

Figure 3 presents the analysis diagram of the produced concentration of hyaluronic acid with mouse fibroblast (3T3) stimulated by 3-O-alkyl ascorbic acid derivatives with the ex vivo process according to this specification and measured in R&D HA-ELISA;

Figure 4 presents the analysis diagram of the produced concentration of hyaluronic acid with human fibroblast (HS68) stimulated by 3-O-alkyl ascorbic acid derivatives with the ex vivo process according to this specification and measured in R&D HA-ELISA; and

Figure 5 presents the analysis diagram of the produced concentration of hyaluronic acid with mouse fibroblast (3T3) stimulated by ascorbic acid (AA) with the ex vivo process according to this specification and measured in R&D HA-ELISA.

## DESCRIPTION OF THE PREFERRED EMBODIMENTS

One preferred embodiment according to this specification discloses an ex vivo process for stimulating the production of hyaluronic acid. According to this embodiment, the mentioned ex vivo process comprises reacting fibroblast cell with 3-O-alkyl ascorbic acid derivatives. The general formula of the mentioned 3-O-alkyl ascorbic acid derivatives is as the following, wherein R1 is H atom and R2 is C2-C10 alkyl group.

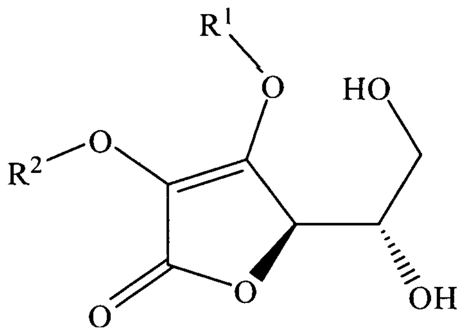

In one preferred example of this embodiment, the mentioned 3-O-alkyl ascorbic acid derivative is 3-O-ethyl-ascorbic acid. According to this example, preferably, the alkyl group of the 3-O-alkyl-ascorbic acid is C2 to C10 alkyl group. In one preferred example of this embodiment, the concentration of the mentioned 3-O-alkyl ascorbic acid derivatives is 7500ppm - 1.25ppm. In another preferred example of this embodiment, the concentration of the mentioned 3-O-alkyl ascorbic acid derivatives is 2500ppm - 1.25ppm.

**Example 1. general procedure of ex vivo stimulating the production of hyaluronic acid with mouse fibroblast:** (hereinafter 3-O-ethyl-ascorbic acid is used for illustration, and this specification is not limited by the following examples)

Materials:

Cell Line: 3T3 (BCRC 60071, purchased from Bioresource Collection and Research Center)

Rinse Buffer: Sterile D-PBS (Dulbecco's phosphate buffered saline, purchased from Corning Incoperated and Caisson Labs)

Complete Medium (as positive control): DMEM medium with 10% Bovine Serum (DMEM purchased from Corning Incoperated and Caisson Labs; BS purchased from Corning Incoperated)

Starvation Medium (as negative control): DMEM medium with 0.1% Bovine Serum (DMEM purchased from Corning Incoperated and Caisson Labs; BS purchased from Corning Incoperated)

Testing material: 3-O-ethyl-ascorbic acid

Hyaluronan Enzyme-Linked Immunosorbent Assay Kit (HA-ELISA) (Brand : Echelon/Cat No : K-1200)

Cell seeding:

Complete medium is used in cell seeding stage. The confluent 3T3 cells are rinsed with sterile D-PBS, and trypsinize all cells and perform cell counting. Then, dilute the cell density to 4E+04/mL and seed 0.5 mL cell liquid (equals to 2E+04 cells) each well in 24 well plates. Incubate 3T3 cells at 37°C, 5% $CO_2$ for about 24 hours.

Controls/testing material treatment:

Starvation medium is used in this stage. The 24-hour-cultured 3T3 cells are rinsed with sterile D-PBS and discarded rinse buffer. Controls and testing materials in different concentrations are respectively added into the 3T3 cells in 24 well plates. Then, the 3T3 cells are incubated at 37°C, with 5% $CO_2$ for about 72 hours.

Quantitation of Hyaluronic Acid:

After incubating for about 72 hours, transfer some supernatant of cells treated with controls and testing materials into new containers. Centrifuge the supernatants at 2000 rpm for 5~10 minutes. The hyaluronic acid content of the centrifuged supernatants are respectively determined by Hyaluronan Enzyme-Linked Immunosorbent Assay Kit, and calculated with the following **Formula 1.**

$$\text{Percentage to Control (\%)} = \frac{\text{HA Content of Positive Control or testing materials}}{\text{HA Content of Negative Control}} \times 100\%$$

## Formula 1

The following **Table** 1 lists several repeat test results according to the above mentioned ex vivo experiments. As shown in **Table 1,** comparing to the negative control, the 2500ppm 3-O-ethyl-ascorbic acid can maximally ex vivo stimulate hyaluronic acid production to 230.6%. It can also be found from **Table 1** that when the concentration of 3-O-ethyl-ascorbic acid is 10 - 5000ppm, the ex vivo stimulated hyaluronic acid production can be efficiently increased by the 3-O-ethyl ascorbic acid. That is, according to this specification, the ex vivo stimulatory of producing hyaluronic acid can be approached efficiently with 3-O-ethyl ascorbic acid.

Due to the cell viability were inhibited by 3-O-ethyl-ascorbic acid with the concentration larger than 7500ppm, this group didn't show ex vivo stimulating ability for hyaluronic acid production. And, 3-O-alkyl ascorbic acid derivatives of those concentrations might become cytotoxicity to the cell so that the ex vivo secretion of hyaluronic acid will be decreased.

**Table 1. Data Analysis of Hyaluronic Acid Content ex vivo Stimulated by 3-O-ethyl-ascorbic acid in mouse fibroblast (3T3)**

| Sample name /concentration | | Entry-1 | Entry-2 | Entry-3 | Entry-4 | Average | Stdev |
|---|---|---|---|---|---|---|---|
| Positive Control | | 796.8% | >max* | 721.8% | 524.0% | 680.8% | 140.9% |
| Negative Control | | 100.0% | 100.0% | 100.0% | 100.0% | 100.0% | 0.0% |
| 3-O-ethyl -ascorbic acid (ppm) | 10000 | 123.0% | 122.4% | 61.1% | 51.0% | 89.4% | 38.7% |
| | 7500 | 64.9% | 123.2% | 120.1% | 55.9% | 91.0% | 35.6% |
| | 5000 | 110.3% | 214.1% | 161.9% | 109.6% | 149.0% | 49.9% |
| | 2500 | 161.6% | 193.4% | 74.2% | 230.6% | 165.0% | 66.7% |
| | 1000 | 216.8% | 212.8% | 80.8% | 85.2% | 148.9% | 76.1% |
| | 100 | 204.5% | 150.2% | 86.0% | ·91.5% | 133.1% | 55.8% |
| | 10 | 203.7% | 197.3% | 92.9% | 164.6% | 164.6% | 50.8% |

* The concentration of sample is out of standard curve.

Figure 1 shows the ex vivo stimulatory effect on Hyaluronic Acid secretion by mouse fibroblast (3T3) of the **Table 1.** From Figure 1, it is obviously that the 3-O-ethyl ascorbic acid can improve the ex vivo stimulating hyaluronic acid production.

**Example 2. general procedure of ex vivo stimulating the production of hyaluronic acid with human fibroblast:** (hereinafter 3-O-ethyl-ascorbic acid is used for illustration, and this specification is not limited by the following examples)

Materials:

Cell Line: Hs68 (BCRC 60038, purchased from Bioresource Collection and Research Center)

Rinse Buffer: Sterile D-PBS (Dulbecco's phosphate buffered saline, purchased from Corning Incoperated and Caisson Labs)

Complete Medium (as positive control): DMEM medium with 10% Fetal Bovine Serum (DMEM purchased from Corning Incoperated and Caisson Labs; FBS purchased from Caisson Labs)

Starvation Medium (as negative control): DMEM medium with 0.1% Fetal Bovine Serum(DMEM purchased from Corning Incoperated and Caisson Labs; FBS purchased from Caisson Labs)

Testing material: 3-O-ethyl-ascorbic acid

Hyaluronan Enzyme-Linked Immunosorbent Assay Kit (HA-ELISA) (Brand: Echelon /Cat No : K-1200)

Cell seeding:

Complete medium is used in cell seeding stage. The confluent Hs68 cells are rinsed with sterile D-PBS, and trypsinize all cells and perform cell counting. Then, dilute the cell density to 4E+04/mL and seed 0.5 mL cell liquid (equals to 2E+04 cells) each well in 24 well plates. Incubate Hs68 cells at 37°C, 5% $CO_2$ for about 24 hours.

Controls/testing material treatment:

Starvation medium is used in this stage. The 24-hour-cultured Hs68 cells are rinsed with sterile D-PBS and discarded rinse buffer. Controls and testing materials in different concentrations are respectively added into the Hs68 cells in 24 well plates. Then, the Hs68 cells are incubated at 37°C, with 5% $CO_2$ for about 72 hours.

Quantitation of Hyaluronic Acid:

After incubating for about 72 hours, transfer some supernatant of cells treating with controls and testing materials into new containers. Centrifuge the supernatants at 2000 rpm for 5~10 minutes. The hyaluronic acid content of the centrifuged supernatants are respectively determined by Hyaluronan Enzyme-Linked Immunosorbent Assay Kit, and calculated with the following **Formula 2.**

$$\text{Percentage to Control (\%)} = \frac{\text{HA Content of Positive Control or testing materials}}{\text{HA Content of Negative Control}} \times 100\%$$

**Formula 2**

The following **Table 2** lists several repeat test results according to the above mentioned ex vivo experiments. As shown in **Table 2,** comparing to the negative control, the 1000ppm 3-O-ethyl-ascorbic acid can maximally ex vivo stimulate hyaluronic acid production to 205.4%. When the concentration of 3-O-ethyl-ascorbic acid is 1000 - 10ppm, it can be found from the mentioned **Table 2** that the ex vivo stimulated hyaluronic acid production is dose-dependently upon the concentration of the 3-O-ethyl ascorbic acid. Additionally, as shown in **Table 2,** according to this specification, the ex vivo stimulatory of producing hyaluronic acid can be approached efficiently with 3-O-ethyl ascorbic acid.

Due to the cell viability were inhibited by 10000ppm 3-O-ethyl-ascorbic acid, this group didn't show dose dependent trend in ex vivo stimulating hyaluronic acid production. Moreover, the ascorbic acid derivatives might be cytotoxicity for producing hyaluronic acid.

**Table 2. Data Analysis of Hyaluronic Acid Content ex vivo Stimulated by 3-O-ethyl-ascorbic acid in human fibroblast (Hs68)**

| Sample Name | Positive Control | Negative Control | 3-O-ethyl -ascorbic acid (ppm) | | | |
|---|---|---|---|---|---|---|
| Sample Concentration | | | 10000 | 1000 | 100 | 10 |
| Entry-1 | 282.2% | 100.0% | 19.4% | 170.1% | 130.3% | 123.3% |
| Entry-2 | 407.3% | 100.0% | 40.6% | 172.6% | 115.1% | 105.7% |
| Entry-3 | 218.7% | 100.0% | 37.4% | 205.4% | 200.9% | 92.7% |
| Average | 302.7% | 100.0% | 32.5% | 182.7% | 148.8% | 107.2% |
| Stdev | 96.0% | 0.0% | 11.4% | 19.7% | 45.8% | 15.3% |

Figure 2 shows the ex vivo stimulatory effect on Hyaluronic Acid secretion by human fibroblast of the **Table 2.** From Figure 2, it can be found that the dose-dependent trend between the 3-O-ethyl ascorbic acid concentration and the ex vivo producing hyaluronic acid is obviously.

**Example 3. ex vivo test of stimulating the production of hyaluronic acid with mouse fibroblast:**

In this example, we try another brand HA-ELISA kit (R&D ; Cat No. DHYAL0) to figure out the reproducibility of the stimulatory effect. Excluding replacing the HA-ELISA kit, the materials and the procedures can be referred to the above **Example 1.**

The following **Table 3** lists several repeat test results according to the above mentioned experiments. As shown in **Table 3,** comparing to the negative control, the 5000ppm 3-O-ethyl-ascorbic acid can maximally ex vivo stimulate hyaluronic acid production to 240.5%. When the concentration of 3-O-ethyl-ascorbic acid is 5000 - 5ppm, it can be found from the **Table 3** that the ex vivo stimulated hyaluronic acid production is dose-dependently upon the concentration of the 3-O-ethyl ascorbic acid. Additionally, referred to **Table 3,** according to this specification, the ex vivo stimulatory of producing hyaluronic acid can be approached efficiently with 3-O-ethyl ascorbic acid.

**Table 3. Data Analysis of Hyaluronic Acid Content ex vivo Stimulated by 3-O-ethyl-ascorbic acid in mouse fibroblast (3T3)**

| Sample name /concentration | | Entry-1 | Entry-2 | Entry-3 | Average | Stdev |
|---|---|---|---|---|---|---|
| Positive Control | | 963.2% | 478.5% | 497.6% | 646.4% | 274.5% |
| Negative Control | | 100.0% | 100.0% | 100.0% | 100.0% | 0.0% |
| 3-O-ethyl -ascorbic acid (ppm) | 10000 | 64.7% | 117.1% | 145.3% | 109.0% | 40.9% |
| | 7500 | 119.9% | 130.8% | 180.7% | 143.8% | 32.4% |
| | 5000 | 131.8% | 180.4% | 240.5% | 184.2% | 54.5% |
| | 2500 | 148.7% | 176.3% | 152.8% | 159.3% | 14.9% |
| | 1000 | 111.0% | 188.3% | 234.1% | 177.8% | 62.2% |
| | 100 | 166.8% | 155.7% | 191.2% | 171.2% | 18.2% |
| | 10 | 101.0% | 158.1% | 182.2% | 147.1% | 41.7% |
| | 7.5 | 115.9% | 143.8% | 148.1% | 135.9% | 17.5% |
| | 5 | 78.2% | 152.4% | 180.6% | 137.1% | 52.9% |
| | 2.5 | 119.7% | 135.2% | 182.8% | 145.9% | 32.9% |
| | 1.25 | 100.0% | 156.8% | 172.0% | 143.0% | 37.9% |

Figure 3 shows the ex vivo stimulatory effect on Hyaluronic Acid secretion by mouse fibroblast (3T3) of the **Table 3.** From Figure 3, it is obviously that the 3-O-ethyl ascorbic acid can improve the ex vivo stimulating hyaluronic acid production, and Hyaluronic Acid ex vivo stimulating effect of the 3-O-ethyl ascorbic acid is obviously, even using kits from different brands.

**Example 4. ex vivo test of stimulating the production of hyaluronic acid with human fibroblast:**

In this example, we try another brand HA-ELISA kit (R&D ; Cat No. DHYAL0) to study the reproducibility of the stimulatory effect. Excluding replacing the HA-ELISA kit, the materials and the procedures can be referred to the above **Example 2.**

The following **Table 4** lists several repeat test results according to the above mentioned experiments. As shown in **Table 4,** comparing to the negative control, the 100ppm 3-O-ethyl-ascorbic acid can maximally ex vivo stimulate hyaluronic acid production to 174.9%. According to **Table 4,** when the concentration of 3-O-ethyl-ascorbic acid is controlled at 1000ppm, 100ppm, and 7.5ppm, the corresponding ex vivo stimulated hyaluronic acid production is 133.9%, 121.9%, and 134.0%. Preferably, when the concentration of 3-O-ethyl-ascorbic acid is 2.5ppm, the ex vivo stimulated hyaluronic acid production still can achieve 136.7%. That is, referred to **Table 4,** the ex vivo stimulatory of producing hyaluronic acid can be approached efficiently with 3-O-ethyl ascorbic acid. It can be found from the **Table 4** that when the concentration of 3-O-ethyl-ascorbic acid is about 1000 - 10ppm, the ex vivo stimulated hyaluronic acid production is dose-dependently upon the concentration of the 3-O-ethyl ascorbic acid.

**Table 4. Data Analysis of Hyaluronic Acid Content ex vivo**

**Stimulated by 3-O-ethyl-ascorbic acid in human fibroblast (HS68)**

| Sample name /concentration | | Entry-1 | Entry-2 | Entry-3 | Entry-4 | Average | Stdev |
|---|---|---|---|---|---|---|---|
| Positive Control | | 410.8% | 359.5% | 342.9% | 198.2% | 327.9% | 91.1% |
| Negative Control | | 100.0% | 100.0% | 100.0% | 100.0% | 100.0% | 0.0% |
| 3-O-ethyl -ascorbic acid (ppm) | 10000 | 50.9% | 32.1% | 37.2% | 42.4% | 40.7% | 8.0% |
| | 7500 | 114.2% | 54.6% | 67.1% | 56.4% | 73.1% | 28.0% |
| | 5000 | 110.0% | 70.6% | 103.2% | 80.3% | 91.0% | 18.6% |
| | 2500 | 99.2% | 96.7% | 78.7% | 140.0% | 103.6% | 25.9% |
| | 1000 | 125.4% | 134.8% | 122.6% | 152.7% | 133.9% | 13.6% |
| | 100 | 123.5% | 104.5% | 84.8% | 174.9% | 121.9% | 38.7% |
| | 10 | 89.1% | 101.2% | 91.2% | 109.5% | 97.7% | 9.4% |
| | 7.5 | 67.9% | 162.0% | 157.3% | 148.8% | 134.0% | 44.4% |
| | 5 | 110.6% | 110.9% | 103.8% | 167.0% | 123.1% | 29.5% |
| | 2.5 | 149.7% | 125.3% | 111.4% | 160.5% | 136.7% | 22.4% |
| | 1.25 | 102.1% | 126.8% | 102.4% | 119.0% | 112.6% | 12.3% |

Figure 4 shows the ex vivo stimulatory effect on Hyaluronic Acid secretion by human fibroblast of the **Table 4.** From Figure 4, it can be found that the dose-dependent trend between the ascorbic acid concentration and the producing hyaluronic acid is obviously even using kits from different Brands.

**Example 5. ex vivo test of stimulating the production of hyaluronic acid with ascorbic acid (AA) in mouse fibroblast:**

In this example, we try to figure out whether the stimulatory effect also can be found on ascorbic acid. Excluding replacing the HA-ELISA kit to another brand HA-ELISA kit (R&D; Cat No. DHYAL0) and replacing the testing material to ascorbic acid, the other materials and the procedures can be referred to the above **Example 1.**

The following **Table 5** lists several repeat ex vivo test results according to the above mentioned ex vivo experiments. As shown in **Table 5,** comparing to the negative control, the 2 - 100ppm ascorbic acid can NOT ex vivo stimulate hyaluronic acid production. Furthermore, in some entries, the mentioned ascorbic acid will decrease the concentration of hyaluronic acid. That is, comparing with the disclosure of this specification, ascorbic acid cannot be employed for the ex vivo stimulatory of producing hyaluronic acid.

**Table 5. Data Anyalysis of Hyaluronic Acid Content ex vivo Stimulated by ascorbic acid in mouse fibroblast (3T3)**

| Sample name /concentration | | Entry-1 | Entry-2 | Entry-3 | Average | Stdev |
|---|---|---|---|---|---|---|
| Positive Control | | 460% | 550% | 647% | 552% | 93% |
| Negative Control | | 100% | 100% | 100% | 100% | 0% |
| Ascorbic Acid (ppm) | 100 | 87% | 93% | 94% | 91% | 4% |
| | 50 | 105% | 100% | 102% | 102% | 2% |
| | 10 | 93% | 108% | 101% | 101% | 7% |
| | 2 | 99% | 64% | 119% | 94% | 27% |

Figure 5 shows the ex vivo stimulatory effect on Hyaluronic Acid secretion by human fibroblast of the **Table 5.** From Figure 5, it can be found that ascorbic acid cannot be helpful to the ex vivo stimulatory of producing hyaluronic acid.

In summary, this application has reported an ex vivo process for ex vivo stimulating the production of hyaluronic acid. According to this application, the mentioned ex vivo process comprises a step of reacting fibroblast cells with 3-O-alkyl ascorbic acid derivatives. The mentioned ex vivo process for ex vivo stimulating the production of hyaluronic acid of this application can stimulate the production of hyaluronic acid of mouse and human fibroblast cell in vitro. More preferably, the ex vivo stimulating production of hyaluronic acid of this application can be approached with few amount 3-O-alkyl ascorbic acid derivatives. That is, this application provides a more safe and efficient ex vivo method for increasing hyaluronic acid in cells.

## Claims

1. An ex vivo process for stimulating the production of hyaluronic acid, comprising:

   a step of reacting fibroblast cells with 3-O-alkyl ascorbic acid derivatives, wherein the general formula of said 3-O-alkyl ascorbic acid derivatives is as the following:

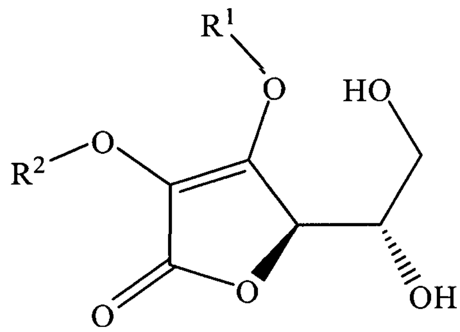

   wherein $R^1$ is H atom, and $R^2$ is C2-C10 alkyl group.

2. The ex vivo process for stimulating the production of hyaluronic acid according to claim 1, wherein the concentration range of said 3-O-alkyl ascorbic acid derivatives is 7500ppm - 1.25ppm.

3. The ex vivo process for stimulating the production of hyaluronic acid according to claim 1, wherein the concentration range of said 3-O-alkyl ascorbic acid derivatives is 2500ppm - 1.25ppm.

4. The ex vivo process for stimulating the production of hyaluronic acid according to claim 1, wherein said 3-O-alkyl ascorbic acid derivatives is 3-O-ethyl-ascorbic acid.

## Patentansprüche

1. Ex vivo-Verfahren zur Stimulation der Erzeugung von Hyaluronsäure, umfassend:

   einen Schritt des Umsetzens von Fibroblastenzellen mit 3-O-Alkylascorbinsäurederivaten, wobei die allgemeine Formel der 3-O-Alkylascorbinsäurederivate wie folgt ist:

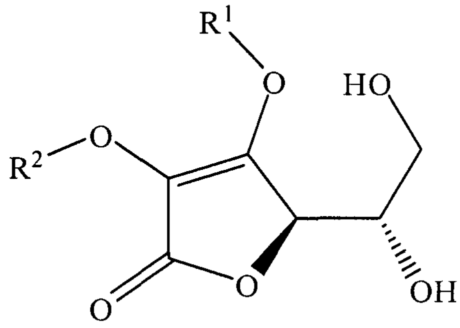

worin $R^1$ ein H-Atom ist und $R^2$ eine C2-C10-Alkylgruppe ist.

2. Ex vivo-Verfahren zur Stimulation der Erzeugung von Hyaluronsäure nach Anspruch 1, bei dem der Konzentrationsbereich der 3-O-Alkylascorbinsäurederivate 7500 ppm bis 1,25 ppm beträgt.

3. Ex vivo-Verfahren zur Stimulation der Erzeugung von Hyaluronsäure nach Anspruch 1, bei dem der Konzentrationsbereich der 3-O-Alkylascorbinsäurederivate 2500 ppm bis 1,25 ppm beträgt.

4. Ex vivo-Verfahren zur Stimulation der Erzeugung von Hyaluronsäure nach Anspruch 1, bei dem es sich bei den 3-O-Alkylascorbinsäurederivaten um 3-O-Ethylascorbinsäure handelt.

## Revendications

1. Procédé *ex vivo* pour stimuler la production d'acide hyaluronique, comprenant :

   une étape consistant à faire réagir des cellules fibroblastiques avec des dérivés d'acide 3-O-alkyl ascorbique, dans laquelle la formule générale desdits dérivés d'acide 3-O-alkyl ascorbique est la suivante :

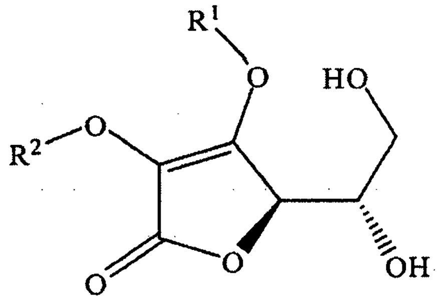

   dans laquelle $R^1$ est un atome de H, et $R^2$ est un groupe alkyle en $C_2$-$C_{10}$.

2. Procédé *ex vivo* pour stimuler la production d'acide hyaluronique selon la revendication 1, dans lequel la gamme de concentration desdits dérivés d'acide 3-O-alkyl ascorbique est de 7500 ppm à 1,25 ppm.

3. Procédé *ex vivo* pour stimuler la production d'acide hyaluronique selon la revendication 1, dans lequel la gamme de concentration desdits dérivés d'acide 3-O-alkyl ascorbique est de 2500 ppm à 1,25 ppm.

4. Procédé *ex vivo* pour stimuler la production d'acide hyaluronique selon la revendication 1, dans lequel ledit dérivé d'acide 3-O-alkyl ascorbique est l'acide 3-O-éthyl-ascorbique.

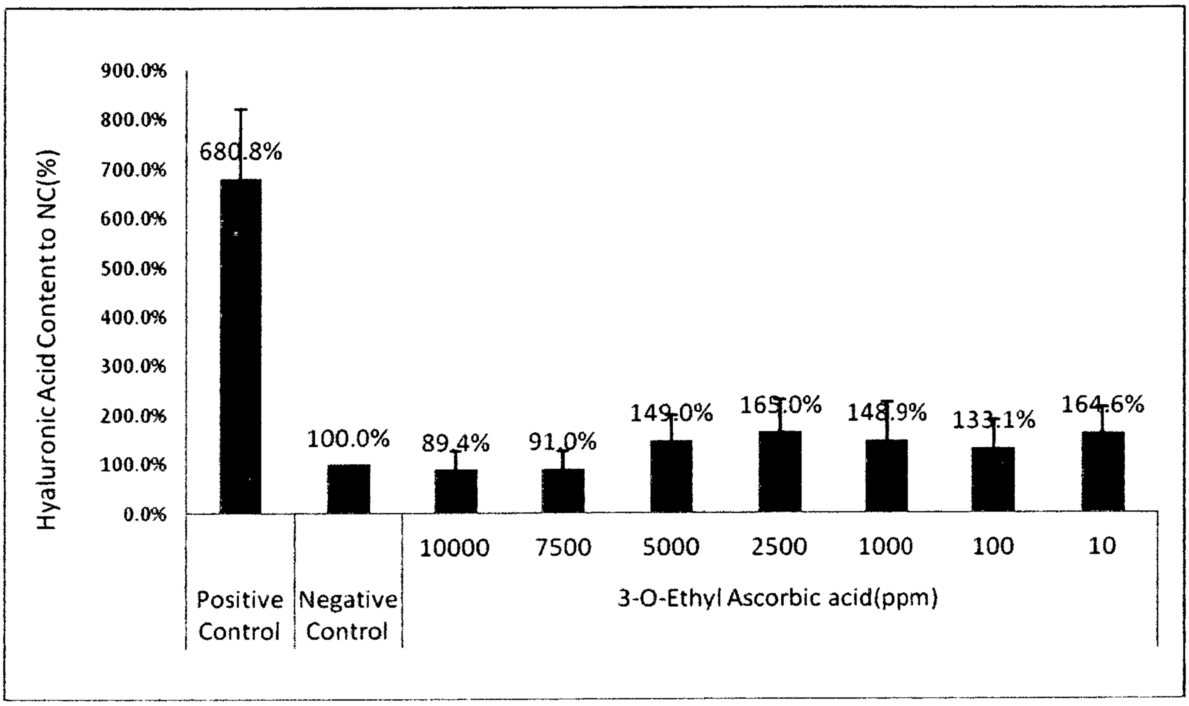


# Figure 1

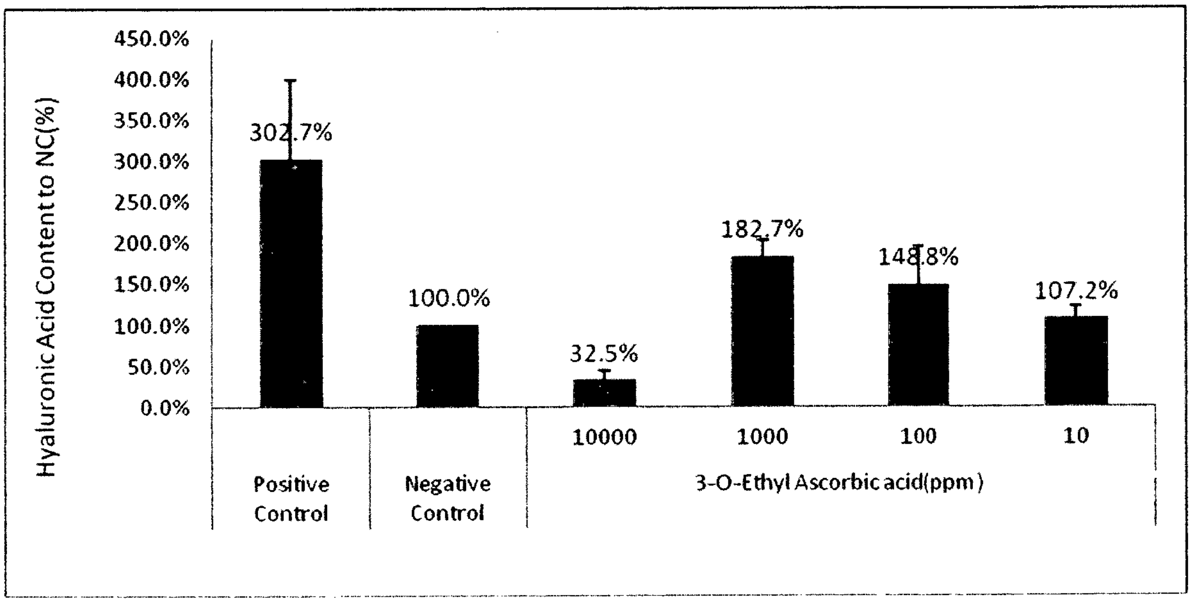


# Figure 2

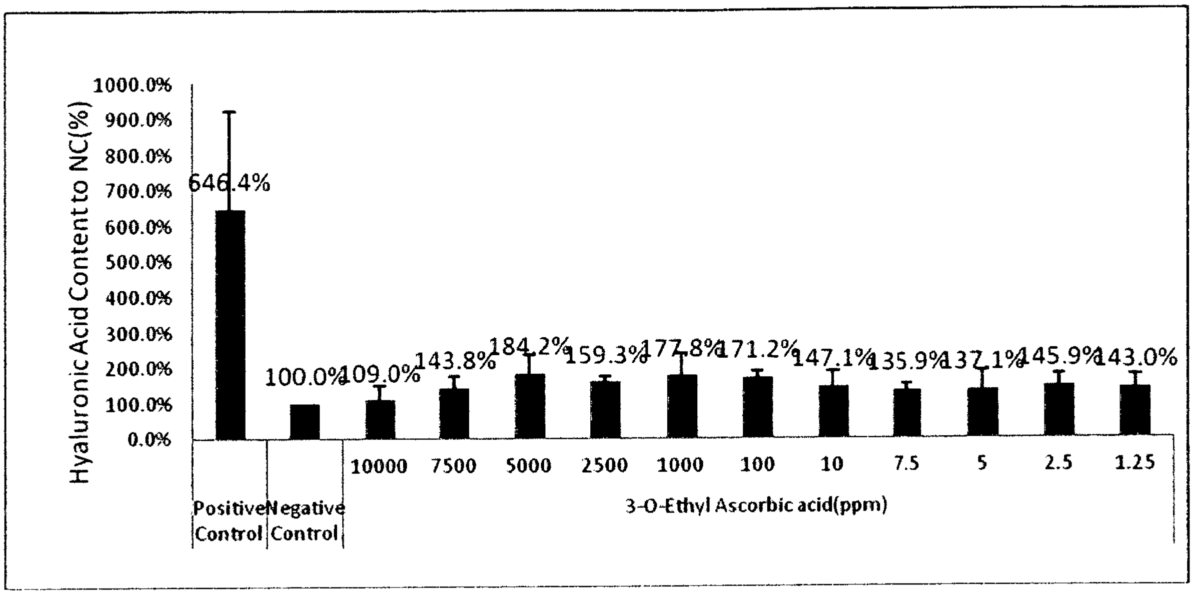


# Figure 3

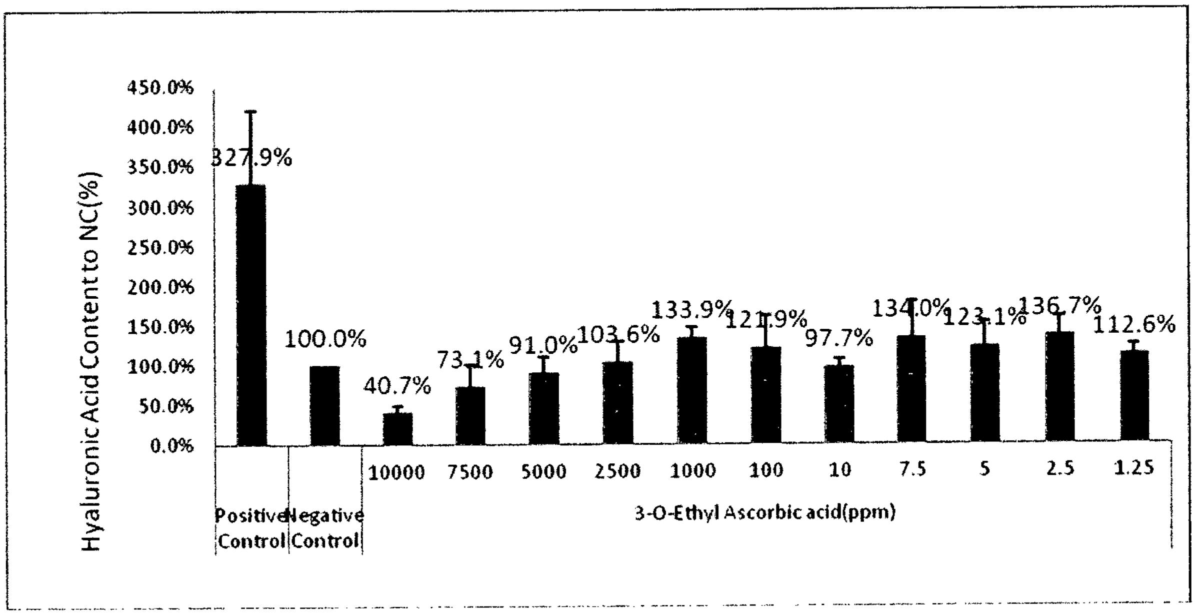


# Figure 4

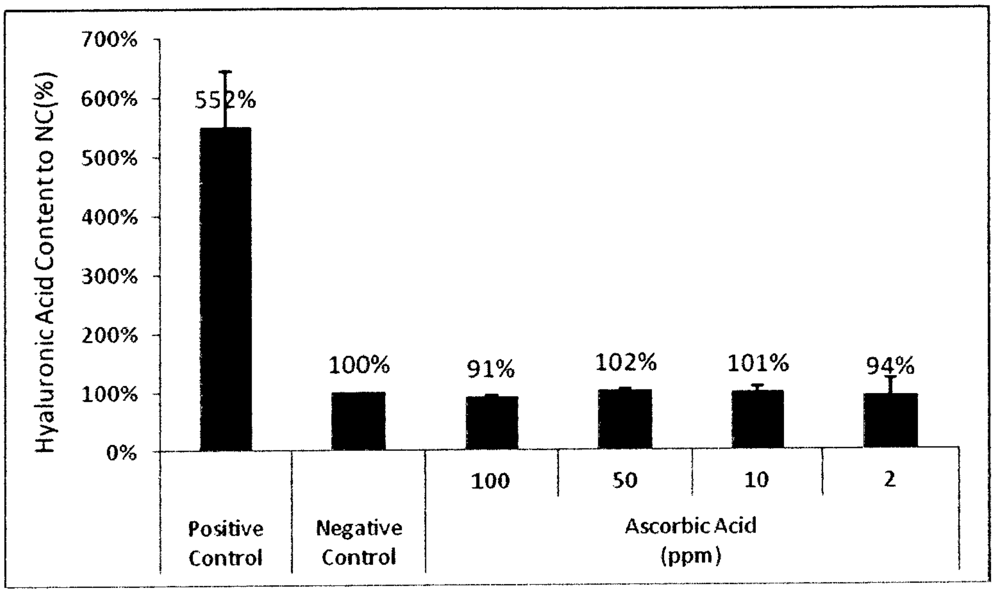
700%
600%
500%
400%
300%
200%
100%
0%
Hyaluronic Acid Content to NC(%)
552%
100%
91%
102%
101%
94%
100
50
10
2
Positive Control
Negative Control
Ascorbic Acid (ppm)

# Figure 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1264593 A2
- JP 2008105985 A